(19) Europäisches Patentamt
European Patent Office
Office européen des brevets

(11) **EP 3 237 046 B1**

(12) **EUROPÄISCHE PATENTSCHRIFT**

(45) Veröffentlichungstag und Bekanntmachung des Hinweises auf die Patenterteilung:
**19.12.2018 Patentblatt 2018/51**

(21) Anmeldenummer: **17718807.5**

(22) Anmeldetag: **16.03.2017**

(51) Int Cl.:
*A61M 13/00* *(2006.01)*     *A61M 16/16* *(2006.01)*

(86) Internationale Anmeldenummer:
**PCT/DE2017/000068**

(87) Internationale Veröffentlichungsnummer:
**WO 2017/157365 (21.09.2017 Gazette 2017/38)**

(54) **INSUFFLATIONSSCHLAUCH FÜR DIE LAPAROSKOPIE MIT HEIZELEMENT, BEFEUCHTUNGSMITTEL UND VORRICHTUNG ZUR BESTIMMUNG DES FEUCHTIGKEITSGEHALTES**

INSUFFLATION HOSE FOR USE IN LAPAROSCOPY, COMPRISING A HEATING ELEMENT, A HUMIDIFYING MEANS AND A DEVICE FOR DETERMINING THE HUMIDITY CONTENT

TUBE D'INSUFFLATION POUR LAPAROSCOPIE AVEC ÉLÉMENT CHAUFFANT, MOYEN D'HUMIDIFICATION, ET DISPOSITIF DE MESURE DU TAUX D'HUMIDITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.03.2016 DE 102016003172**

(43) Veröffentlichungstag der Anmeldung:
**01.11.2017 Patentblatt 2017/44**

(73) Patentinhaber: **W.O.M. World of Medicine GmbH 10587 Berlin (DE)**

(72) Erfinder:
• **KÖTH, Yves**
**12683 Berlin (DE)**
• **MENZEL, Felix**
**13088 Berlin (DE)**

(74) Vertreter: **Jungblut & Seuss Patentanwälte Max-Dohrn-Strasse 10 10589 Berlin (DE)**

(56) Entgegenhaltungen:
WO-A1-2013/137753     WO-A1-2014/111083
WO-A1-2015/135040     WO-A2-2015/027980
DE-A1-102013 000 489     DE-A1-102013 000 492
US-A1- 2003 181 857

## Beschreibung

Die vorliegende Erfindung betrifft einen Insufflator mit Insufflationsschlauch mit integriertem Heizelement und Befeuchtungsmittel für die Laparoskopie, wobei der Heizdraht zur Widerstandsmessung ausgelegt ist und einen Nachfüllalarm ermöglicht.

Hintergrund und Stand der Technik

Die Laparoskopie ist ein medizinischer Eingriff, bei dem die Bauchhöhle und die darin liegenden Organe visuell überprüft werden können. Hierzu werden üblicherweise kleine Hautschnitte (0,3 bis 2 Zentimeter) in die Bauchdecke gemacht und durch diese ein Trokar eingebracht, welcher wiederum eine optische Vorrichtung aufnehmen kann. Mithilfe eines speziellen Endoskops (Laparoskop) kann der Bauchraum eingesehen werden. Bei der diagnostischen Laparoskopie wird der Bauchraum lediglich visuell inspiziert, im Rahmen einer therapeutischen Laparoskopie können auch operative Eingriffe vorgenommen werden.

Üblicherweise wird zu Beginn der Laparoskopie zunächst der Bauchraum mit Gas befüllt, um ein Pneumoperitoneum zu schaffen. Hierzu sind bereits verschiedene Gase verwendet worden, wie z. B. Luft, Stickstoff oder Kohlendioxid ($CO_2$). Die Verwendung von Kohlendioxidgas hat sich besonders gut bewährt. Es wurde festgestellt, dass es, insbesondere bei längeren laparoskopischen Eingriffen sinnvoll ist, das eingeführte Gas einerseits zu erwärmen und anderseits zu befeuchten. Die Gaserwärmung dient dazu, den Patienten nicht abzukühlen, sowie ein diffuses Schmerzgefühl des Patienten zu vermeiden, welches wahrscheinlich eine Folge lokaler Abkühlung infolge des Eintritts von kaltem Gas ist. Die Befeuchtung dient dazu, einem Austrocknen der inneren Bauchoberflächen vorzubeugen, um die dabei entstehende Abkühlung zu vermeiden.

Hierzu sind im Stand der Technik bereits Anregungen gegeben. So beschreibt beispielsweise die deutsche Patentschrift DE 19510710 eine Vorrichtung, die ein Mittel zur Anpassung der Gasfeuchte vorsieht (beispielsweise einen Schwamm) und welche optional ein zusätzliches Heizelement enthalten kann.

Die DE 10 2013 000492 A1 beschreibt einen Schlauch mit integriertem Heizelement für die Laparoskopie, welcher gleichzeitig ein Befeuchtungsmittel enthält. Gemäß dieser Druckschrift wird das Befeuchtungsmittel vor einer Operation mit Wasser befeuchtet. Je nach Wasseraufnahme des dort beschriebenen Materials, dem Gasvolumenstrom und der Dauer der Operation kann intraoperativ eine Nachbefeuchtung des Befeuchtungsmittels nötig sein. Da die Verdunstungsrate des Wassers, von einer Anzahl von Parametern abhängt, kann bislang nur geschätzt werden, wann eine Nachfüllung möglich ist. Alternativ werden Ausführungen beschrieben, die einen Feuchtesensor zum Ermitteln der Gasfeuchte im Gaskanal anordnen. Damit verbinden sich allerdings mehrere Nachteile. Zum einen muss der Feuchtesensor elektrisch angebunden werden, was das Design der Filterschnittstelle komplizierter macht. Weiterhin erzeugt der Feuchtesensor einen nicht unerheblichen Strömungswiderstand im Gaskanal. Dieser führt zu einer geringeren Flussrate, die den aktuellen Flowanforderungen widerspricht.

Eine weitere Vorrichtung zur Befeuchtung von Gasen in der Medizintechnik wird in der DE 3617031A1 (Prioritäten: NZ 21263, NZ 215123 und NZ 214694) beschrieben. Im Rahmen eines aufwändig herzustellenden Schlauchsystems ist ein stets wassergefüllter Schlauch vorgesehen. Über eine mikroporöse Schlauchwand wird Wasserdampf an das Gas abgegeben. Ein Sensor überwacht die Wassertemperatur.

Die Aufgabe der vorliegenden Erfindung ist es, den Zustand des Befeuchtungsmittels in Bezug auf seinen Wassergehalt, d.h. den Wassergehalt des Befeuchtungsmittels zuermitteln, ohne die oben genannten Nachteile zu implementieren. Primäre Zielstellung ist die Generierung eines Nachfüllalarms/-signals, d. h. eines Signals, welches dem Benutzer mitteilt, wann eine Nachfüllung von Wasser nötig ist. Im Rahmen der vorliegenden Erfindung werden die Begriffe "Wassergehalt des Befeuchtungsmittels" und "Feuchtigkeit des Befeuchtungsmittels" als synonym angesehen.

## Lösung der Aufgabe

Die Lösung dieser Aufgabe erfolgt durch den Gegenstand der Patentansprüche, d. h. eine Insufflationseinrichtung mit Insufflationsschlauch, der seinerseits eine Heiz- und Befeuchtungseinrichtung aufweist. Die Messung des Wassergehalts des Befeuchtungsmittels erfolgt durch die Auswertung der Widerstandsmessung des Heizdrahtes.

Die Erfindung betrifft daher eine Insufflationseinrichtung zur Verwendung in der Medizintechnik enthaltend

einen Insufflator zur Gasversorgung und einen Insufflationsschlauch,
wobei der Insufflationsschlauch in seinem Inneren ein Befeuchtungsmaterial enthält, wobei das Befeuchtungsmaterial Kontakt mit einem Heizelement hat,
wobei das Heizelement durch Anlegen eines Stroms aktivierbar ist,
wobei das Heizelement aus einem Draht besteht, wobei der Draht seinen Widerstand mit der Temperatur ändert, wobei der Insufflator eine Vorrichtung zur Messung des Drahtwiderstandes enthält, dadurch gekennzeichnet, dass der Insufflator eine Rechenvorrichtung enthält, die aus der gemessenen Widerstandsänderung des Heizdrahtes während des Heizvorgangs, den Wassergehalt des Befeuchtungsmaterials bestimmt.

Wie eingangs beschrieben, besteht die erfindungsgemäße Einrichtung zunächst aus einem Insufflator und einem Heizschlauch. Der Heizschlauch ist gemäß der Lehre der DE 10 2013 000492 A1 ausgestaltet, d. h.

im Schlauchinneren befindet sich ein Befeuchtungsmaterial in dessen unmittelbarer Nähe ein Heizelement positioniert ist. Das Heizelement besteht dabei aus einem Heizdraht, der in bevorzugter Weise in Form einer Drahtwendel ausgeführt ist. Dieser Heizdraht kann sich im Innern des Schlauches befinden. Eine alternative Ausführungsform besteht darin, dass der Heizdraht in die Schlauchwand eingearbeitet ist. Der Heizdraht hat üblicherweise eine Länge von 50 Zentimetern bis 10 Metern. Der Drahtdurchmesser beträgt typischerweise 0,25 bis 2 Millimeter. Auf diese Weise kann eine Heizleistung von 5 bis 50 Watt erzielt werden. Wenn der Draht im Inneren des Schlauches positioniert ist, dann ist er in bevorzugter Weise in Form einer Wendel mit einem Durchmesser von 3 bis 4 Millimeter ausgeführt.

In unmittelbarer Nähe des Heizelementes, bevorzugterweise in direktem Kontakt, wird ein Befeuchtungsmaterial positioniert. Es handelt sich dabei um ein poröses Material, welches in der Lage ist, Flüssigkeit, insbesondere Wasser, aufzunehmen. Dieses Befeuchtungsmaterial kann beispielsweise die oben geschilderte Drahtwendel umschließen. Für den Fall, dass der Draht in die Schlauchwand eingearbeitet ist, ist es bevorzugt, dass das Befeuchtungsmaterial direkten Kontakt mit der Schlauchwand aufweist. Als Befeuchtungsmaterial kann im einfachsten Fall sterile Baumwolle verwendet werden, die in der Lage ist, eine gewisse Menge Wasser aufzunehmen. Alternativ können Schwämme, superabsorbierende Polymere (SAP), Löschpapier oder Material aus Phenolharzen verwenden werden. Alternative Ausführungsformen sind denkbar.

Entscheidend für die erfindungsgemäße Verwendung ist es, dass für den Heizdraht ein Material verwendet wird, dessen Widerstand sich mit der Temperatur ändert. Eine derartige Widerstandsänderung bei Temperaturänderungen des Heizdrahtes wird physikalisch über den Temperaturkoeffizienten definiert. Wünschenswert für den Heizdraht ist ein Material, bei dem der Widerstand zwischen 0 Grad und 100 Grad Celsius linear mit der Temperatur ansteigt, wobei die Steigerung ausreichend messbar ist. Wenn der Temperaturkoeffizient des Materials nicht linear verläuft, wird die Auswertung aufwendiger, ist aber dennoch realisierbar. Wünschenswert ist ein Widerstandsanstieg im genannten Temperaturbereich von 0,1 Ohm/K. Derartige Materialien sind beispielsweise Eisen, Nickel oder Legierungen hiervon. Derartige Produkte können auf dem Markt beschafft werden und benötigen an dieser Stelle keine weitere Erläuterung. Typische Heizdrähte für den erfindungsgemäßen Einsatz haben einen Widerstand von 2 bis 30 Ohm/m und weisen einen Durchmesser von 0,25 bis 2 Millimeter auf. Für den Fachmann auf dem Gebiet ist klar, dass diese Zusammensetzung des Drahtes und dessen Durchmesser über die gesamte Länge möglichst konstant sein müssen.

Ein derartiger Heizdraht von z. B. 6 Metern Länge und einem Widerstand von 3 Ohm/m kann in einem 3 Meter langen Schlauch integriert werden. Ist die Leistung des Drahtes pro Meter höher oder niedriger (wegen eines abweichenden Widerstandswertes), kann über eine Änderung der Drahtlänge die benötigte Heizleistung variiert werden.

Die Verwendung eines derartigen Drahtes erlaubt es, wie in der DE 10 2013 000489 A1 geschildert, die Temperatur des Drahtes ohne einen zusätzlichen Fühler zu messen. Hierzu wird der Widerstand des Drahtes gemessen und hieraus die Drahttemperatur berechnet. Beispiele hierfür sind der erwähnten Druckschrift DE 10 2013 000489 A1 genannt.

Um den Wassergehalt des Befeuchtungsmittels zu bestimmen, wird erfindungsgemäß das zeitliche Widerstandsverhalten des Heizdrahtes während des Heizvorgangs betrachtet. Bedingt durch die unterschiedlichen Eigenschaften des Heizsystems mit feuchtem Befeuchtungsmittel gegenüber trockenem Befeuchtungsmittel ändert sich die zeitliche Charakteristik der Widerstandsänderung des Heizdrahtes infolge einer elektrischen Anregung. Dieses Verhalten kann sowohl in der Heizphase einer Heizperiode als auch in einer Abkühlungsphase einer Heizperiode analysiert werden, um den Wassergehalt zu bestimmen. Der Widerstand des Heizdrahtes steigt je nach Wassergehalt des Befeuchtungsmittels infolge einer Anregung unterschiedlich schnell und unterschiedlich stark an. Bei einer plötzlich auftretenden und daraufhin andauernden konstanten Heizleistung erreicht der Heizdraht nach einer bestimmten Zeit, welche von der Systemfeuchtigkeit abhängig ist, ein thermisches Gleichgewicht, sodass der Widerstand nach Ablauf dieser "Aufheizzeit" nicht weiter ansteigt. Der Wert $T_{100}$ beschreibt die Zeitkonstante des Heizsystems, welche der Zeit entspricht, in der 100% des stationären Endwertes erreicht sind. Im einfachsten Fall kann die Messung dieser Zeit $T_{100}$ zur Erreichung dieses stationären Endwertes als Aussage über den Wassergehalt herangezogen werden. Wird die Heizleistung ausgehend von diesem stationären Endwert plötzlich wieder deaktiviert, geht das System wieder in seinen Ausgangszustand zurück, wobei die "Abkühlungszeit" zum Erreichen dieses Ausgangszustands ab dem Zeitpunkt der Deaktivierung der Heizung ebenfalls von dem Feuchtegrad abhängt. Unter gleichen Bedingungen entspricht auch diese Zeit der Zeit $T_{100}$.

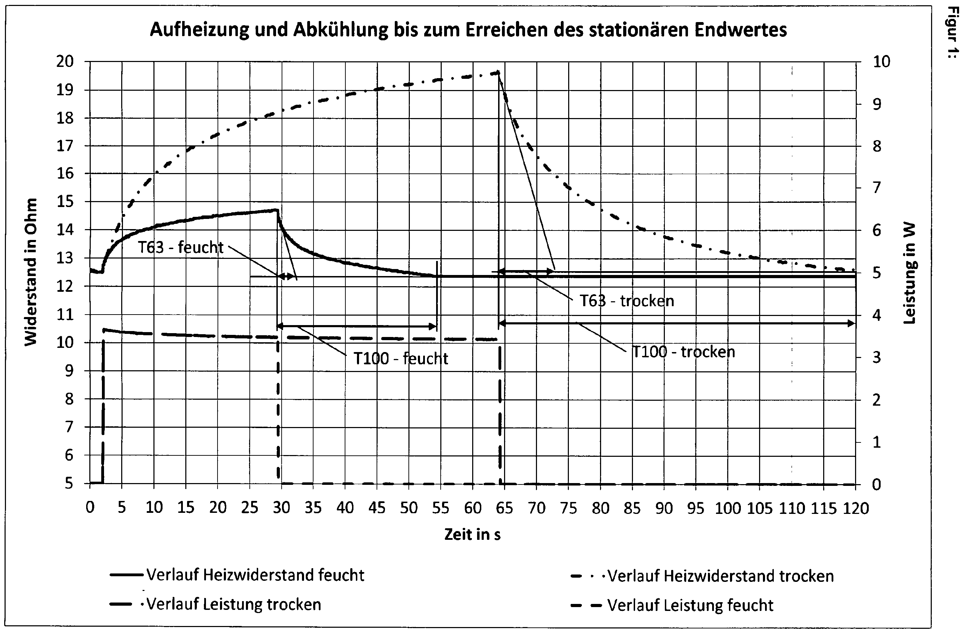

Figur 1 zeigt einen Vergleich der Aufheizung und Abkühlung bis zum Erreichen des stationären Endwertes bei feuchtem und trockenem Befeuchtungsmittel.

Bis das thermische Gleichgewicht erreicht ist kann unterschiedlich viel Zeit vergehen. Deswegen kann es sinnvoll sein, auch aus kurzen und unterschiedlich langen "Heizimpulsen", die z.B. bei der Regelung mit einem Zweipunktregler auftreten, den Befeuchtungsgrad zu bestimmen. Hierzu ließe sich beispielweise die Änderungsrate des Widerstandes über der Zeit auswerten. Dieses Verfahren wäre jedoch fehleranfällig, da diese Änderungsrate von verschiedenen Faktoren, bspw. der Heizleistung abhängig ist und im Falle einer Regelung nicht gewährleistet werden kann, dass die Heizleistung immer einen bestimmten Wert annimmt. Des Weiteren ist die Widerstandsänderungsrate zu verschiedenen Zeiträumen während des Aufheizens oder Abkühlens wie in den Verläufen in Figur 1 dargestellt ist nicht konstant, sodass genaue "Triggerpunkte" definiert werden müssten.

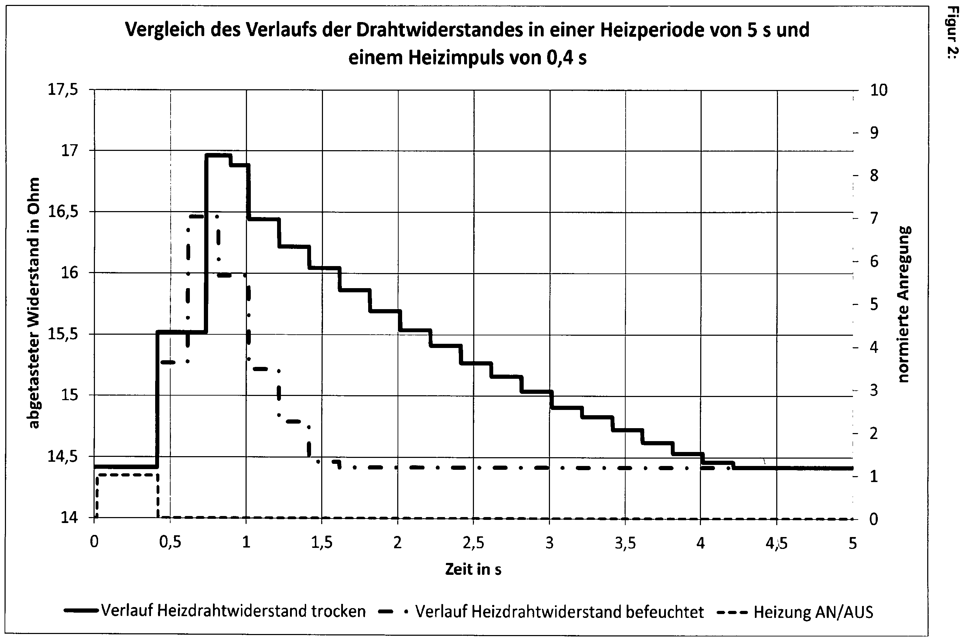

Figur 2 zeigt einen Vergleich des Verlaufes des Drahtwiderstandes bei kurzen Heizimpulsen bei feuchtem und trockenem Befeuchtungsmittel.

Im Folgenden wird ein weiteres erfindungsgemäßes Verfahren dargestellt, welches beliebig kurze Heiz- und Abkühlphasen erlaubt und dabei hinreichend genau und fehlertolerant den Befeuchtungsgrad ermitteln kann. Das Verfahren besteht darin, die zeitlichen Verläufe des Drahtwiderstandes in Abhängigkeit von der zeitlichen Anregung durch ein Modell zu beschreiben und die Parameter dieses Modells zur Laufzeit zu identifizieren.

Das Modell enthält dazu sowohl die Anregung als auch den Widerstand des Drahtes. Dieses Modell kann bspw. eine lineare Differentialgleichung erster Ordnung sein:

$$T_{63} \cdot \dot{R}_H(t) + R_H(t) = K \cdot S_H(t)$$

$R_H$ = Widerstand des Heizdrahtes
$K$ = Verstärkungsfaktor
$S_H$ = Zustand der Heizung (ON/OFF)

In dem Modell beschreibt $T_{63}$ die Zeitkonstante des Heizsystems, welche sofern das Modell zutrifft, der Zeit entspricht, in der 63% des stationären Endwertes erreicht sind. Der Parameter $K$ beschreibt die sogenannte stationäre Verstärkung. Mit einem geeigneten Algorithmus, bspw. einem rekursiven "Least-Square-Verfahren", kann eine diskrete Formulierung dieses Modells an die abgetasteten Messwerte der Anregung und des Drahtwiderstandes durch die schrittweise Optimierung der Parameter $T_{63}$ und $K$ angepasst werden. Ähnlich der Berechnung eines gleitenden Mittelwertes kann dieser Algorithmus während der Laufzeit auf gemessene Verläufe, wie beispielhaft in Figur 1 oder Figur 2 dargestellt, angewandt werden. Weitergehende Informationen zur Parameteridentifikation linearer Systeme finden sich z.B. in Isermann, Mechatronische Systeme Grundlagen, 2. Aufl., Kapitel 7.2 Parameterschätzung für zeitdiskrete Signale, S. 339-343.

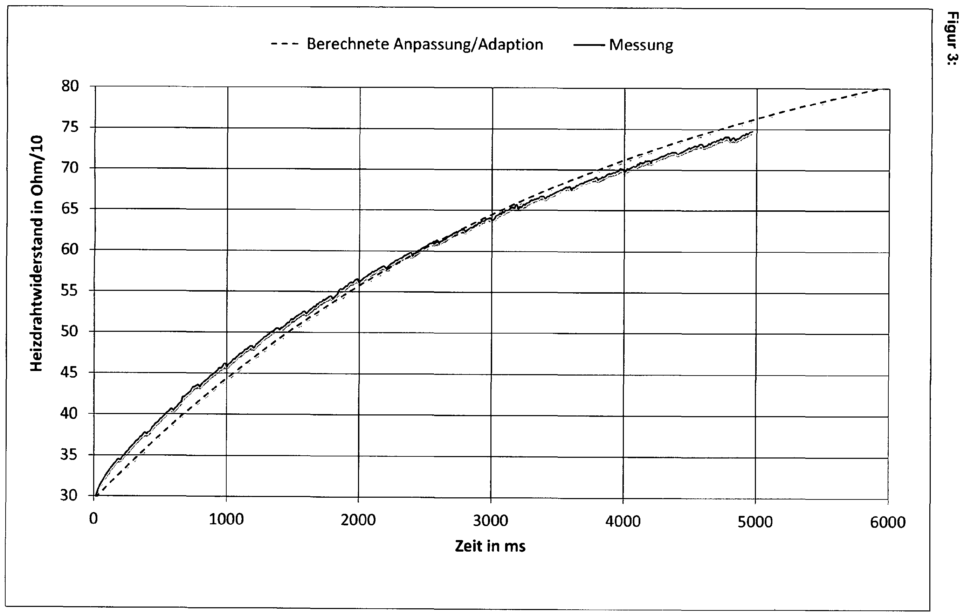

Figur 3 zeigt die Adaption des Modells an die Messung innerhalb von 5 s.

Durch das beschriebene Verfahren ist die Ermittlung von $T_{63}$ auch aus kurzen dynamischen Verläufen, wie in Figur 2 dargestellt, und somit ein Rückschluss auf den Wassergehalt des Befeuchtungsmittels möglich.

Die Werte von $T_{63}$ für die Ermittlung des Wassergehaltes nach dem dargestellten Verfahren hängen von der genauen Schlauchspezifikation ab. Es ist daher sinnvoll, jede unterschiedliche Ausführungsform eines erfindungsgemäßen Schlauches auszumessen und die gemessenen Werte dann zur Grundlage der Einstellung des Insufflators zu machen. Wie bereits in der Druckschrift DE 10 2013 000489 A1 beschrieben, erfolgt die Anpassung der Heizleistung an den tatsächlichen Bedarf (in Abhängigkeit von Außentemperatur und Gasvolumenstrom) durch Verlängerung oder Verkürzung der Heizzyklen. Für die erfindungsgemäße Verwendung kann es daher erforderlich sein, spezielle Messzyklen zur Messung des Befeuchtungsgrades des Befeuchtungsmittels zu definieren und in die beschriebene Regelung kurzzeitig einzugreifen. So könnte beispielsweise ein Mal pro Minute in einem gesonderten Messintervall eine fünfsekündige Heizphase mit genau definierter Heizleistung erfolgen. Andere Gestaltungen der Messzyklen sind ohne Weiteres denkbar. In Analogie zu der Beschreibung in der DE 10 2013 000489 A1 ist es natürlich möglich, die genauen Kennwerte des individuellen Heizschlauchs (Temperaturkoeffizient im Bereich von 0 bis 100 Grad Celsius, Abhängigkeit der Gas-Temperatur am Schlauchausgang von Heizleistung und Gasvolumenstrom, Aufheizraten in Abhängigkeit von Feuchtigkeit des Befeuchtungsmittels) im Rahmen der Produktion des Schlauches zu messen und auf einen Flash-Speicher zu schreiben, der (beispielsweise) am maschinenseitigen Schlauchanschluss positioniert ist. Bei Anschluss des Schlauches an das Insufflationsgerät können so beispielsweise die Daten an das Gerät übertragen und zur weiteren Verwendung bereitgestellt werden.

Im Rahmen des klinischen Einsatzes kann davon ausgegangen werden, dass sich die Umgebungsparameter (Temperatur, Luftdruck, Gasfeuchtigkeit am Schlaucheingang weder während einer Operation, noch von Operation zu Operation ändern.

Die Erfindung betrifft daher auch ein Verfahren zur Messung des Wassergehaltes eines Befeuchtungsmaterials, welches sich in einem Insufflationsschlauch einer Insufflationseinrichtung gemäß Anspruch 1 befindet, welcher von einem Gas durchströmt wird, dadurch gekennzeichnet, dass

    a) der Heizdraht in Intervallen beheizt wird,
    b) der Widerstand des Heizdrahtes zu mindestens zwei Zeitpunkten während des Heizintervalls gemessen wird,
    c) dass aus den ermittelten Widerständen eine Widerstandsänderung, eine Widerstandsänderungszeit oder eine Zeitkonstante $T_{100}$, $T_{63}$ berechnet wird, wobei $T_{63}$ der Zeit entspricht, in der 63% der stationären Endtemperatur erreicht werden, und wobei $T_{100}$ der Zeit entspricht, in der 100% der stationären Endtemperatur erreicht werden, und
    d) dass aus der Auswertung der Widerstandsänderung, der Widerstandsänderungszeit oder der Zeitkonstanten der Wassergehalt des Befeuchtungsmittels bestimmt wird.

In einer speziellen Ausgestaltung des Verfahrens wird die benötigte Zeit zur Änderung des Drahtwiderstandes um einen definierten Wert (z.B. einer Widerstandsänderung, die einer Temperaturänderung von 0,1°C, 0,5°C, 1°C oder 2°C entspricht) nach Aktivierung oder Deaktivierung des Heizstroms zur Bestimmung des Wassergehaltes des Befeuchtungsmediums verwendet.

In einer weiteren Ausgestaltung des Verfahrens wird die Änderung des Drahtwiderstandes nach Aktivierung oder Deaktivierung des Heizstroms für eine definierte Dauer (z.B. 0,1 s, 0,5s, 1s, 2 s oder 5s) zur Bestimmung des Wassergehaltes des Befeuchtungsmediums verwendet wird.

Mit dem erfindungsgemäßen Verfahren kann die Feuchtigkeit des Befeuchtungsmittels erstmals mit der erforderlichen Genauigkeit ohne einen zusätzlichen Feuchtesensor gemessen werden. Auf diese Weise ist es möglich, ein Alarmsignal auszulösen, wenn die Feuchtigkeit des Befeuchtungsmittels einen voreingestellten Schwellenwert unterschreitet. Der voreingestellten Schwellenwert kann zum Beispiel 50%, 40%, 30%, 20 %, 10% oder 5% der maximalen Feuchtigkeit betragen. Nach der Auslösung des Alarmsignals kann das medizinische Bedienpersonal beispielsweise Wasser nachfüllen.

Der Fachmann auf dem Gebiet kann alternative und/oder ergänzende Ausführungsformen der Erfindung realisieren, ohne erfinderisch tätig werden zu müssen.

**Patentansprüche**

1. Insufflationseinrichtung zur Verwendung in der Medizintechnik enthaltend
einen Insufflator zur Gasversorgung und einen Insufflationsschlauch,
wobei der Insufflationsschlauch in seinem Inneren ein Befeuchtungsmaterial enthält,
wobei das Befeuchtungsmaterial Kontakt mit einem Heizelement hat,
wobei das Heizelement durch Anlegen eines Stroms aktivierbar ist,
wobei das Heizelement aus einem Draht besteht,
wobei der Draht seinen Widerstand mit der Temperatur ändert,
wobei der Insufflator eine Vorrichtung zur Messung des Drahtwiderstandes enthält,
**dadurch gekennzeichnet, dass**
der Insufflator eine Rechenvorrichtung enthält, die aus der gemessenen Widerstandsänderung des Heizdrahtes während des Heizvorgangs, den Wassergehalt des Befeuchtungsmaterials bestimmt.

2. Vorrichtung gemäß Anspruch 1, **dadurch gekennzeichnet, dass** die benötigte Zeit $T_{100}$ zur Änderung des Drahtwiderstandes auf seinen stationären Endwert nach Aktivierung oder Deaktivierung des Heizstroms zur Bestimmung des Wassergehaltes des Befeuchtungsmediums verwendet wird.

3. Vorrichtung gemäß Anspruch 1, **dadurch gekennzeichnet, dass** der aktuell ermittelte Heizdrahtwiderstand gemeinsam mit dem Zustand der Heizung ON/OFF in einem mathematischen Algorithmus verarbeitet wird, wobei als Ergebnis eine Zeitkonstante $T_{63}$ berechnet wird, wobei $T_{63}$ der Zeit entspricht, in der 63% der stationären Endtemperatur erreicht werden,
wobei die Zeitkonstante $T_{63}$ als Maß für den Wassergehalt des Befeuchtungsmaterials dient.

4. Vorrichtung gemäß Anspruch 1, 2, oder 3, **dadurch gekennzeichnet, dass** der ermittelte Wassergehalt zur Anzeige des Wassergehaltes und als Alarm zum Nachfüllen des Befeuchtungsmediums verwendet wird.

5. Vorrichtung gemäß Anspruch 1 bis 4, **dadurch gekennzeichnet, dass** für den einzelnen Schlauch ein Satz Kalibrierdaten auf einem Datenträger abgelegt sind, welcher an dem Schlauch angebracht ist.

6. Verfahren zur Messung des Wassergehaltes eines Befeuchtungsmaterials, welches sich in einem Insufflationsschlauch einer Insufflationseinrichtung gemäß Anspruch 1 befindet, welcher von einem Gas durchströmt wird,
**dadurch gekennzeichnet, dass**

a) der Heizdraht in Intervallen beheizt wird,
b) der Widerstand des Heizdrahtes zu mindestens zwei Zeitpunkten während des Heizintervalls gemessen wird,
c) dass aus den ermittelten Widerständen eine Widerstandsänderung, eine Widerstandsänderungszeit oder eine Zeitkonstante $T_{100}$, $T_{63}$ berechnet wird,

wobei $T_{63}$ der Zeit entspricht, in der 63% der stationären Endtemperatur erreicht werden und wobei $T_{100}$ der Zeit entspricht, in der 100% der stationären Endtemperatur erreicht werden,
und

d) dass aus der Auswertung der Widerstandsänderung, der Widerstandsänderungszeit oder der Zeitkonstanten der Wassergehalt des Befeuchtungsmaterials bestimmt wird.

7. Verfahren gemäß Anspruch 6, **dadurch gekennzeichnet, dass** die benötigte Zeit zur Änderung des Drahtwiderstandes um einen definierten Wert nach Aktivierung oder Deaktivierung des Heizstroms zur Bestimmung des Wassergehaltes des Befeuchtungsmaterials verwendet wird.

8. Verfahren gemäß Anspruch 6, **dadurch gekennzeichnet, dass** die Änderung des Drahtwiderstandes nach Aktivierung oder Deaktivierung des Heizstroms für eine definierte Dauer zur Bestimmung des Wassergehaltes des Befeuchtungsmaterials verwendet wird.

9. Verfahren gemäß Anspruch 6, **dadurch gekennzeichnet, dass** ein Alarmsignal ausgelöst wird, wenn der Wassergehalt des Befeuchtungsmaterials einen voreingestellten Schwellenwert unterschreitet.

10. Verfahren gemäß Anspruch 9, **dadurch gekennzeichnet, dass** der voreingestellte Schwellenwert 50%, 40%, 30%, 20%, 10% oder 5% der maximalen Feuchtigkeit entspricht.

**Claims**

1. An insufflation device for use in medical engineering, including an insufflator for gas supply and an insufflation hose,
the insufflation hose including, in its interior, a humectant material,
the humectant material being in contact with a heating element,
the heating element being activatable by applying a current,
the heating element being made of a wire, the wire resistance being changed with the temperature,
the insufflator including a device for measurement of the wire resistance,
**characterized by** that
the insufflator includes a computing device determining, from the measured resistance change of the heating wire during the heating process, the water content of the humectant material.

2. The device according to claim 1, **characterized by** that the required time $T_{100}$ for changing the wire resistance to its stationary final value after activation or deactivation of the heating current is used for determination of the water content of the humectant medium.

3. The device according to claim 1, **characterized by** that the actually determined heating wire resistance is processed commonly with the state of the heating ON/OFF in a mathematical algorithm, as a result a time constant $T_{63}$ being calculated, wherein $T_{63}$ corresponds to the time, in which 63 % of the stationary final temperature are achieved, wherein the time constant $T_{63}$ serves as a measure for the water content of the humectant material.

4. The device according to claim 1, 2 or 3, **characterized by** that the determined water content is used for indication of the water content and as a warning signal for refilling the humectant medium.

5. The device according to claim 1 to 4, **characterized by** that for the individual hose a set of calibration data is stored on a data carrier that is attached at the hose.

6. A method for measurement of the water content of a humectant material, which is present in an insufflation hose of an insufflation device according to claim 1, and which is passed by a gas, **characterized by** that

   a) the heating wire is heated in intervals,
   b) the resistance of the heating wire is measured at least at two points of time during the heating interval,
   c) from the determined resistances, a resistance change, a resistance change time, or a time constant $T_{100}$, $T_{63}$ is calculated,

   wherein $T_{63}$ corresponds to the time, in which 63 % of the stationary final temperature are achieved and wherein $T_{100}$ corresponds to the time, in which 100 % of the stationary final temperature are achieved, and

   d) that from the evaluation of the resistance change, of the resistance change time or of the time constants, the water content of the humectant material is determined.

7. The method according to claim 6, **characterized by** that the required time for changing the wire resistance by a defined value after activation or deactivation of the heating current is used for determination of the water content of the humectant material.

8. The method according to claim 6, **characterized by** that the change of the wire resistance after activation or deactivation of the heating current for a defined duration is used for determination of the water content of the humectant material.

9. The method according to claim 6, **characterized by** that a warning signal is initiated, when the water content of the humectant material falls below a preset threshold value.

10. The method according to claim 9, **characterized by** that the preset threshold value corresponds to 50 %, 40 %, 30 %, 20 %, 10 % or 5 % of the maximum humidity.

## Revendications

**1.** Dispositif d'insufflation pour l'utilisation dans la technique médicale, comportant un insufflateur pour alimentation en gaz et un tube d'insufflation,

le tube d'insufflation comportant en son intérieur un matériau d'humidification,

le matériau d'humidification étant en contact avec un élément de chauffage,

l'élément de chauffage étant activable par l'application d'un courant,

l'élément de chauffage étant compris d'un fil, la résistance du fil étant changée avec la température,

l'insufflateur comportant un dispositif pour mesurer la résistance du fil,

**caractérisé en ce que**

l'insufflateur comporte un dispositif de calcul déterminant, à partir de la variation de la résistance mesurée du fil de chauffage pendant le procédé de chauffage, la teneur en eau du matériau d'humidification.

**2.** Dispositif selon la revendication 1, **caractérisé en ce que** le temps $T_{100}$ nécessaire à la variation de la résistance du fil à sa valeur finale stationnaire après l'activation ou la désactivation du courant de chauffage est utilisé pour la détermination de la teneur en eau du milieu d'humidification.

**3.** Dispositif selon la revendication 1, **caractérisé en ce que** la résistance du fil de chauffage actuellement déterminée est traitée conjointement à l'état du chauffage ON/OFF dans un algorithme mathématique, comme résultat une constante de temps $T_{63}$ étant calculée, où $T_{63}$ correspond au temps, dans lequel 63 % de la température finale stationnaire sont obtenus,

dans lequel la constante de temps $T_{63}$ sert d'une mesure pour la teneur en eau du matériau d'humidification.

**4.** Dispositif selon la revendication 1, 2 ou 3, **caractérisé en ce que** la teneur en eau déterminée est utilisée pour visualiser la teneur en eau et comme alerte pour un rechargement du milieu d'humidification.

**5.** Dispositif selon la revendication 1 à 4, **caractérisé en ce que** pour le tube individuel un jeu de données de calibration est enregistré sur un support qui est attaché au tube.

**6.** Procédé de mesurage de la teneur en eau d'un matériau d'humidification, qui est présent dans un tube d'insufflation d'un dispositif d'insufflation selon la revendication 1, et qui est traversé par un gaz, **caractérisé en ce que**

a) le fil de chauffage est chauffé selon des intervalles,

b) la résistance du fil de chauffage est mesurée au moins à deux instants pendant l'intervalle de chauffage,

c) à partir des résistances déterminées, une variation de la résistance, un temps de la variation de la résistance, ou une constante de temps $T_{100}$, $T_{63}$ est calculée,

dans lequel $T_{63}$ correspond au temps, dans lequel 63 % de la température finale stationnaire sont obtenus et dans lequel $T_{100}$ correspond au temps, dans lequel 100 % de la température finale stationnaire sont obtenus, et

d) qu'à partir de l'évaluation de la variation de la résistance, du temps de la variation de la résistance ou de la constante de temps, la teneur en eau du matériau d'humidification est déterminée.

**7.** Procédé selon la revendication 6, **caractérisé en ce que** le temps nécessaire à la variation de la résistance du fil par une valeur définie après l'activation ou la désactivation du courant de chauffage est utilisé pour la détermination de la teneur en eau du matériau d'humidification.

**8.** Procédé selon la revendication 6, **caractérisé en ce que** la variation de la résistance du fil après l'activation ou la désactivation du courant de chauffage pendant une durée définie est utilisée pour la détermination de la teneur en eau du matériau d'humidification.

**9.** Procédé selon la revendication 6, **caractérisé en ce qu'**un signal d'alerte est provoqué, si la teneur en eau du matériau d'humidification est inférieure à une valeur de seuil prédéterminée.

**10.** Procédé selon la revendication 9, **caractérisé en ce que** la valeur de seuil prédéterminée correspond à 50 %, 40 %, 30 %, 20 %, 10 % ou 5 % de l'humidité maximale.

Figur 1:

Figur 2:

EP 3 237 046 B1

**Vergleich des Verlaufs der Drahtwiderstandes in einer Heizperiode von 5 s und einem Heizimpuls von 0,4 s**

——Verlauf Heizdrahtwiderstand trocken  — · Verlauf Heizdrahtwiderstand befeuchtet  ——— Heizung AN/AUS

**Figur 3:**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19510710
- DE 102013000492 A1
- DE 3617031 A1
- NZ 21263
- NZ 215123
- NZ 214694
- DE 102013000489 A1


**In der Beschreibung aufgeführte Nicht-Patentliteratur**

- Parameterschätzung für zeitdiskrete Signale. **ISERMANN.** Mechatronische Systeme Grundlagen. 339-343